(19) Europäisches Patentamt
European Patent Office
Office européen des brevets

(11) **EP 3 377 889 B1**

(12) **EUROPÄISCHE PATENTSCHRIFT**

(45) Veröffentlichungstag und Bekanntmachung des Hinweises auf die Patenterteilung:
**02.06.2021 Patentblatt 2021/22**

(21) Anmeldenummer: **16784483.6**

(22) Anmeldetag: **19.10.2016**

(51) Int Cl.:
*G01N 31/12* *(2006.01)*    *G01N 33/00* *(2006.01)*

(86) Internationale Anmeldenummer:
**PCT/EP2016/075098**

(87) Internationale Veröffentlichungsnummer:
**WO 2017/084825 (26.05.2017 Gazette 2017/21)**

(54) **ANALYSEGERÄT UND VERFAHREN**

ANALYSIS DEVICE AND METHOD

APPAREIL D'ANALYSE ET PROCÉDÉ

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **19.11.2015 DE 102015120095**

(43) Veröffentlichungstag der Anmeldung:
**26.09.2018 Patentblatt 2018/39**

(73) Patentinhaber: **Analytik Jena GmbH 07745 Jena (DE)**

(72) Erfinder:
• **EHRLING, Christiane**
**98693 Ilmenau (DE)**
• **HENNEBERG, Heiko**
**99338 Plaue (DE)**
• **KNÖFEL, Robert**
**98693 Ilmenau (DE)**
• **SCHEIDE, Kristin**
**98693 Ilmenau (DE)**

(74) Vertreter: **Andres, Angelika Maria Endress+Hauser Group Services (Deutschland) AG+Co. KG Colmarer Straße 6 79576 Weil am Rhein (DE)**

(56) Entgegenhaltungen:
EP-A2- 0 989 401      DE-A1-102008 013 754
DE-T2- 69 228 519      DE-U1-202012 102 724
DE-U1-202013 105 594

EP 3 377 889 B1

## Beschreibung

Die Erfindung betrifft ein Analysegerät zur Bestimmung einer von der Konzentration eines oder mehrerer Inhaltsstoffe einer Probe abhängigen Messgröße.

Eine solche Messgröße kann beispielsweise eine Konzentration einer oder mehrerer Substanzen oder eines Elements, z.B. Schwefel, Chlor oder Wasserstoff, in der Probe oder ein Summenparameter wie der gesamte organisch gebundene Kohlenstoffgehalt (Total Organic Carbon, Abk. TOC), ein Gesamtgehalt an Kohlenstoff (organisch und anorganisch gebunden, Abk. TC) oder der gesamte gebundene Stickstoff (Total Nitrogen, Abk. $TN_b$) sein.

Bekannte Analysegeräte zur automatisierten Bestimmung solcher Messgrößen umfassen einen Aufschlussreaktor, in den eine feste, flüssige oder gasförmige Probe eindosiert wird. In dem Aufschlussreaktor, der beispielsweise als Pyrolyserohr ausgestaltet sein kann, werden die Inhaltsstoffe der Probe thermisch aufgeschlossen. Dabei wird organisch und anorganisch gebundener Kohlenstoff zu Kohlendioxid $CO_2$, Stickstoff zu Stickstoffoxid $NO_x$, Schwefel zu Schwefeloxiden $SO_2/SO_3$, Chlor zu Chlorwasserstoff $HCl$ und Wasserstoff zu Wasser $H_2O$ umgesetzt. Das dabei entstehende Gas bzw. Gasgemisch wird mit Hilfe eines permanent den Aufschlussreaktor durchströmenden Trägergases, das in der Regel auch den für die Umsetzung nötigen Sauerstoff liefert, durch eine Trocknungs- und Absorbereinheit einer Messeinrichtung zugeführt, die zur Bestimmung der Messgröße dient.

In EP 989 401 A2 ist ein Analysegerät zur Elementaranalyse von Kohlenstoff, Wasserstoff, Stickstoff, Schwefel und Sauerstoff beschrieben, das einen Aufschlussreaktor und ein System zur Zuleitung einer abgemessenen Sauerstoffmenge in den Aufschlussreaktor aufweist. Zur Durchführung einer Analyse wird dem Aufschlussreaktor eine bekannte und vorgegebene Sauerstoffmenge zugeführt, die mittels eines Reservoirs abgemessen wird. Um die Sauerstoffmenge einzustellen muss das Aufnahmevermögen des Reservoirs geändert werden. Mit einem Trägergasstrom wird das abgemessene Sauerstoffvolumen aus dem Reservoir in den Aufschlussreaktor transportiert.

In DE 692 28 519 T2 ist ein Verfahren und eine Vorrichtung zu Elementaranalyse von Halogenen, Schwefel und Phosphor beschrieben, bei dem die Probe und ein Sauerstoff enthaltender Gasstrom einem Verbrennungs-Oxidations-Reaktor zugeführt werden, um die Probenbestandteile zu oxidieren. In einer Ausgestaltung kann der Sauerstoff auch als Trägergas wirken, alternativ wird eine Menge Sauerstoff, die ausreichend ist, um die Probenoxidation auszuführen, zuerst in einem Dosier- und Speichermittel, wie eine Schleife mit bekanntem Volumen, gespeichert und danach dem Reaktor zugeführt. In diesem letzteren Fall ist das Trägergas ein Edelgas, z.B. Helium.

In DE 10 2008 013754 A1 ist ein Messverfahren und eine Vorrichtung zur Bestimmung des Gehalts eines chemischen Elements, z.B. Kohlenstoff, Stickstoff oder Phosphor, oder eines Wasserqualitätsparameters, z.B. des Gesamt-Sauerstoffbedarfs, in Frischwasser oder Abwasser beschrieben. Eine Probe des Frisch- oder Abwassers wird in einem sauerstoffhaltigen Trägergasstrom einer Reaktion zugeführt und das Reaktionsprodukt zu Bestimmung des Element-Gehalts oder des Parameters analysiert. Der Sauerstoffanteil des Trägergases wird über eine ventilgesteuert dosierte Sauerstoff- oder Lufteinleitung in einen Strom eines inerten Trägergasbestandteils und/oder durch ventilgesteuerte Einleitung des inerten Bestandteils in einen Sauerstoff- oder Luftstrom eingestellt. Die Zuführung des so erzeugten Trägergasstroms zum Reaktor kann über einen Pufferspeicher zum Ausgleich von Druckschwankungen erfolgen.

In einigen Anwendungen, insbesondere für die Analytik im Spurenbereich, wird als Reaktions- und Trägergas hochreiner Sauerstoff verwendet. Die Messeinrichtung umfasst ein oder mehrere spezifische Detektoren, die dazu dienen, den Anteil der für die zu bestimmende Messgröße relevanten Oxidationsprodukte in dem dem Detektor zugeleiteten Trägergasstrom zu ermitteln. Soll beispielsweise der TOC-Wert der Probe ermittelt werden, dient als spezifischer Detektor ein Infrarot-Detektor, der den $CO_2$-Gehalt des Trägergasstroms bestimmt, aus dem ein Messwert des TOC-Werts der Probe abgeleitet werden kann. Zur $TN_b$-Bestimmung kann ein $NO_x$-Gehalt des Gasstroms mittels einer Chemilumineszenz-Messung bestimmt werden.

Aus DE 20 2013 105 594 U1 ist ein Analysator zur Bestimmung der Zusammensetzung von Materialproben bekannt. Der Analysator weist eine Verbrennungseinrichtung mit einer Brennkammer auf, in der eine zu analysierende Materialprobe positionierbar ist, wobei die Brennkammer einen Sauerstoffeinlass, der an eine Sauerstoffquelle anschließbar ist, und einen Verbrennungsgasauslass aufweist. Die Brennkammer besitzt einen Verbrennungsgasauslass, über den sie mit einer Verbrennungsgas-Sammelkammer verbunden ist, die zwischen der Wandung eines Verbrennungsgas-Sammelbehälters und einem in dem Sammelbehälter verschiebbaren, gegenüber der Behälterwandung abgedichteten Kolben gebildet ist. Aufgrund der sich in der Verbrennungsgas-Sammelkammer sammelnden Verbrennungsgase wird der Kolben nach oben gedrückt. Ein Auslass der Verbrennungsgas-Sammelkammer ist mit mehreren Detektoren verbunden, über welche die Bestandteile des Verbrennungsgases analysiert werden können. Nach abgeschlossener Verbrennung wird der Kolben über einen Antrieb niedergedrückt, so dass die Verbrennungsgase durch den Auslass zu den Detektoren gelangen.

Die Bereitstellung von Sauerstoff als Trägergas bzw. als Reaktionspartner für den thermischen Aufschluss der Proben erfolgt in herkömmlichen Analysegeräten üblicherweise über Druckgasflaschen, Gasgeneratoren oder mittels Adsorbentien. Nachteilig bei diesen Verfahren ist dabei der erforderliche Kosten- und Geräteaufwand sowie bei der Verwendung von Adsorbentien der geringe Reinheitsgrad des Sauerstoffs.

Aus DE 20 2012 102 724 U1 ist ein Analysegerät mit einem thermischen Aufschlusssystem bekannt geworden, das eine Einrichtung zur direkten Erzeugung von Sauerstoff aus Umgebungsluft umfasst. Hierzu dient ein keramisches Material mit Perowskitstruktur, das beispielsweise als Membran oder Granulat ausgebildet sein kann, und das bei hohen Temperaturen eine Sauerstofffionen-Leitfähigkeit aufweist. Diese Sauerstoffionen-Leitfähigkeit erlaubt eine Abtrennung des Sauerstoffs von den übrigen Bestandteilen der Umgebungsluft, indem Sauerstoff selektiv durch das keramische Material transportiert wird. Um das keramische Material auf die für den Sauerstofftransport erforderliche Temperatur zu heizen, wird bei dem in DE 20 2012 102 724 U1 beschriebenen Analysegerät die Prozesswärme des thermischen Aufschlusssystems ausgenutzt.

Die zum Betreiben des Analysegeräts benötigte Sauerstoffmenge kann über die Betriebsdauer des Analysegeräts schwanken. Beispielsweise kann das Analysegerät neben dem Aufschlusssystem weitere Sauerstoffverbraucher umfassen, die diskontinuierlich, d.h. nur während bestimmter Betriebsphasen, Sauerstoff benötigen. Beispielsweise kann ein Analysegerät zur TOC-Bestimmung eine Vorrichtung zum Abtrennen und/oder Bestimmen des anorganisch gebundenen Kohlenstoffs aufweisen, die dazu ausgestaltet ist, den anorganisch gebundenen Kohlenstoff vor dem Aufschluss aus der angesäuerten Probe mit Sauerstoff auszublasen.

Bei der Elementaranalyse wird der Probe vorzugsweise nur die für den thermischen Aufschluss der in der Probe enthaltenen Verbindungen des nachzuweisenden Elements benötigte Sauerstoffmenge in einem Trägergasstrom zugeführt. In diesem Fall ist es wünschenswert, die zugesetzte Sauerstoffmenge möglichst genau steuern zu können.

Die in DE 202012 102 724 U1 beschriebene Sauerstofferzeugungseinrichtung stellt typischerweise eine im Wesentlichen als Funktion der Zeit konstante Menge an Sauerstoff bereit. Diese ergibt sich aus der Dimensionierung des zur Sauerstofferzeugung verwendeten keramischen Materials und der Betriebstemperatur der Einrichtung. Um zu gewährleisten, dass dem Analysegerät jederzeit eine ausreichende Menge an Sauerstoff zur Verfügung steht, müsste das keramische Material so dimensioniert werden, dass kontinuierlich die maximal benötigte Menge an Sauerstoff, ggfs. mit einer zusätzlichen Sicherheitsmarge, zur Verfügung steht. Andererseits ist es wünschenswert, die Sauerstofferzeugungseinrichtung möglichst kompakt auszugestalten und die benötigte Menge an keramischem Material so niedrig wie möglich zu halten.

Es ist nun die Aufgabe der Erfindung, das aus dem Stand der Technik bekannte Analysegerät so weiterzubilden, dass die Sauerstofferzeugungseinrichtung kompakt und materialsparend ausgestaltet werden kann.

Diese Aufgabe wird erfindungsgemäß gelöst durch das Analysegerät gemäß Anspruch 1 und das Verfahren gemäß Anspruch 12. Vorteilhafte Ausgestaltungen der Erfindung sind in den abhängigen Ansprüchen angegeben.

Das erfindungsgemäße Analysegerät zur Bestimmung einer von der Konzentration eines oder mehrerer Inhaltsstoffe einer Probe abhängigen Messgröße, umfasst:

- einen Aufschlussreaktor mit einem Gaseinlass und einem Gasauslass;
- eine Messeinrichtung, die mit dem Gasauslass des Aufschlussreaktors verbunden ist;
- eine Kontroll- und Auswerteeinrichtung, welche dazu ausgestaltet ist, von der Messeinrichtung registrierte, von der Messgröße abhängige Messwerte zu empfangen und zu verarbeiten;
- eine Sauerstofferzeugungseinrichtung; und
- ein Gasleitungssystem, welches die Sauerstofferzeugungseinrichtung mit dem Gaseinlass des Aufschlussreaktors verbindet, um dem Aufschlussreaktor von der Sauerstofferzeugungseinrichtung erzeugten Sauerstoff zuzuführen,

wobei das Gasleitungssystem eine Gasspeichereinrichtung umfasst, welche mindestens eine zur Aufnahme einer zu speichernden Sauerstoffmenge dienende Kammer mit einem variablen Volumen aufweist.

Die Gasspeichereinrichtung dient zur Aufnahme des kontinuierlich von der Sauerstofferzeugungseinrichtung erzeugten Sauerstoffs. Die Sauerstoff-Aufnahmekapazität der Gasspeichereinrichtung ist vorteilhafterweise so auf die pro Zeiteinheit erzeugte Sauerstoffmenge abgestimmt, dass sie in Betriebsphasen des Analysegeräts, in denen die aktuell erzeugte Sauerstoffmenge nicht vollständig verbraucht wird, eine Sauerstoffmenge speichern kann, die groß genug ist, dass in Betriebsphasen, in denen mehr als die aktuell erzeugte Sauerstoffmenge benötigt wird, eine ausreichende Menge an Sauerstoff zur Verfügung steht. Auf diese Weise ist es möglich, die Sauerstofferzeugungseinrichtung und insbesondere das zur Sauerstofferzeugung dienende Material der Sauerstofferzeugungseinrichtung kompakt auszugestalten.

In einer Ausgestaltung umfasst das Gasleitungssystem eine Steuerungseinrichtung zur Flussregulierung mindestens eines durch das Gasleitungssystem von der Gasspeichereinrichtung zum Aufschlussreaktor strömenden Sauerstoffstroms.

In die Kammer der Gasspeichereinrichtung kann eine Gaszuleitung münden, welche, insbesondere über eine Pumpe, mit einem Gasauslass der Sauerstofferzeugungseinrichtung verbunden ist, wobei die Kammer einen Gasauslass aufweist, welcher, insbesondere über die Steuerungseinrichtung, mit dem Gaseinlass des Aufschlussreaktors verbunden ist.

In einer weiteren Ausgestaltung verbindet das Gasleitungssystem die Sauerstofferzeugungseinrichtung über die Gasspeichereinrichtung zusätzlich mit einem oder mehreren weiteren Verbrauchern, um diesen von der Sauerstofferzeugungseinrichtung erzeugten Sauerstoff zuzuführen und wobei die Steuerungseinrichtung zur Flussregulierung zusätzlich dazu ausgestaltet ist, einen Transport von Sauerstoff durch das Gasleitungssystem von der Gasspeichereinrichtung zu dem einen oder den mehreren weiteren Verbrauchern zu steuern.

Ein weiterer Verbraucher kann für den Fall, dass es sich bei dem Analysegerät um ein Gerät zur Bestimmung des Parameters TOC handelt, beispielsweise eine Einrichtung zur Entfernung von anorganisch gebundenem Kohlenstoff sein.

Der Gasauslass der mindestens einen Kammer der Gasspeichereinrichtung kann über die Steuerungseinrichtung mit dem einen weiteren Verbraucher oder mit einem oder mehreren, insbesondere mit jedem, der weiteren Verbraucher verbunden sein.

In einer Ausgestaltung, in der in die Kammer eine Gaszuleitung mündet, welche mit einem Gasauslass der Sauerstofferzeugungseinrichtung verbunden ist, kann die Kammer einen Wandungsbereich umfassen, welcher dazu ausgestaltet ist, bei Zunahme einer in der Kammer enthaltenen Sauerstoffmenge unter Vergrößerung des Volumens der Kammer gegen eine Rückstellkraft ausgelenkt zu werden. In dieser Ausgestaltung ist eine Speicherung von Sauerstoff durch Auslenkung des Wandungsbereichs möglich. Bei erhöhter Sauerstoffentnahme aus der Kammer mittels der Steuereinrichtung zur Flussregulierung wird der ausgelenkte Wandungsbereich durch die Rückstellkraft unter Verkleinerung des Volumens der Kammer wieder in seine ursprüngliche Position zurückbewegt. In dieser Ausgestaltung werden somit keine weiteren Mittel, wie z.B. Antriebe, zur aktiven Vergrößerung oder Verkleinerung des Kammervolumens zur Aufnahme bzw. Abgabe von Sauerstoff benötigt.

Die Gasspeichereinrichtung kann beispielsweise durch einen auf einer Seite durch einen Boden verschlossenen Zylinder und einen in dem Zylinder axial bezüglich der Zylinderachse beweglichen Kolben gebildet sein, wobei die axiale Position des Kolbens von der in der Kammer enthaltenen Sauerstoffmenge abhängt. Der Kolben kann den Zylinder auf dessen dem Boden gegenüberliegenden Seite gasdicht verschließen. Beispielsweise kann der Kolben innerhalb des Zylinders gasdicht geführt sein. Es ist auch möglich, dass der Kolben mittels eines Faltenbalgs oder mittels einer flexiblen Membran mit der Wandung des Zylinders gasdicht verbunden ist, so dass der Zylinder gasdicht verschlossen ist und gleichzeitig eine axiale Beweglichkeit des Kolbens gewährleistet ist. In dieser Ausgestaltung dient als Rückstellkraft, gegen die der Kolben unter Vergrößerung des Kammervolumens ausgelenkt wird, die auf den Kolben wirkende Gewichtskraft. Ist der Kolben mittels einer Membran oder eines Faltenbalgs mit der Zylinderwandung verbunden, kann alternativ oder zusätzlich zur Gewichtskraft des Kolbens auch die Elastizität der Membran oder des Faltenbalgs eine auf den Kolben wirkende Rückstellkraft bewirken.

Das Gasleitungssystem kann Mittel zur Überwachung der Auslenkung des Wandungsbereichs der Kammer, insbesondere der axialen Position des Kolbens, umfassen. Solche Mittel können beispielsweise Näherungsschalter, Lichtschranken, bildverarbeitende Sensoren oder Wegsensoren sein, die ihre mit der axialen Position des Kolbens korrelierenden Messsignale an die Kontroll- und Auswerteeinrichtung des Analysesystems ausgeben. Die axiale Position des Kolbens lässt sich in eine in der Kammer gespeicherte Sauerstoffmenge umrechnen. Die Überwachung der axialen Position des Kolbens erlaubt es somit, vor Beginn einer Betriebsphase des Analysegeräts zu überprüfen, ob eine für diese Betriebsphase benötigte Sauerstoffmenge zur Verfügung steht.

Die Kontroll- und Auswerteeinrichtung des Analysegeräts kann dazu ausgestaltet sein, die axiale Position des Kolbens repräsentierende Messsignale zu empfangen und aus diesen die aktuell im Gasspeicher enthaltene Sauerstoffmenge zu ermitteln. Anhand dieser kann sie eine Bearbeitungsphase des Analysesystems mit erhöhtem Sauerstoffbedarf starten oder, falls die im Gasspeicher aktuell vorliegende Sauerstoffmenge nicht ausreicht, verzögern.

Um eine größere Sauerstoffmenge speichern zu können, kann die Gasspeichereinrichtung in dieser Ausgestaltung auch mehrere gleichartig ausgestaltete derartige Kammern aufweisen. In diesem Fall kann jede der Kammern über eine steuerbare Ventileinrichtung mit dem Gasauslass der Sauerstofferzeugungseinrichtung verbunden sein.

Die Gasspeichereinrichtung kann in einer alternativen Ausgestaltung mindestens zwei Kammern aufweisen, deren Volumen mittels einer Steuerung veränderbar ist, wobei in jede der Kammern eine Gaszuleitung mündet, welche mit dem Gasauslass der Sauerstofferzeugungseinrichtung verbindbar ist, und wobei jede der Kammern einen Gasauslass aufweist, welcher, insbesondere über die Steuerungseinrichtung zur Flussregulierung, mit dem Gaseinlass des Aufschlussreaktors und/oder gegebenenfalls vorhandenen weiteren Verbrauchern verbunden ist. In dieser Ausgestaltung kann das Volumen der Kammern aktiv mittels der Steuerung verändert werden, was die Einstellung eines geregelten Über- oder Unterdrucks in den Kammern erlaubt. Mittels des einstellbaren Über- oder Unterdrucks in den Kammern kann ein Durchfluss des in die Kammern transportierten oder aus den Kammern entnommenen Sauerstoffs eingestellt werden. Dies kann beispielsweise dazu ausgenutzt werden, eine vorgegebene Sauerstoffmenge zu dosieren und einem Verbraucher oder dem Aufschlussreaktor zuzuführen.

In dieser Ausgestaltung kann jede der Gaszuleitungen über eine Ventilbaugruppe mit dem Gasauslass der Sauerstofferzeugungseinrichtung verbunden sein, wobei die Ventilbaugruppe dazu ausgestaltet ist, vom Gasauslass der Sauerstofferzeugungseinrichtung zu den Kammern führende Gasleitungen wahlweise, insbesondere abwechselnd oder nach einem von der Steuerung vorgegebenen Ablauf, zu sperren oder freizugeben.

Die mindestens zwei Kammern können jeweils durch einen auf einer Seite durch einen Boden verschlossenen Zylinder und einen in dem Zylinder axial bezüglich der Zylinderachse beweglichen Kolben gebildet sein, wobei jeder Kolben mit einem eine axiale Bewegung des Kolbens bewirkenden, insbesondere von der Steuerung betätigbaren, Antrieb verbunden ist. Der Kolben kann den jeweiligen Zylinder auf seiner dem Boden gegenüberliegenden Seite gasdicht verschließen, beispielsweise kann er innerhalb des Zylinders gasdicht geführt sein. Es ist auch möglich, dass der Kolben mittels eines Faltenbalgs oder mittels einer flexiblen Membran mit der Wandung des Zylinders gasdicht verbunden ist, so dass der Zylinder gasdicht verschlossen ist und gleichzeitig eine axiale Beweglichkeit des Kolbens gewährleistet ist.

Die Gasspeichereinrichtung kann in dieser Ausgestaltung Drucksensoren umfassen, welche dazu ausgestaltet sind, den in den Kammern herrschenden Druck zu erfassen und entsprechende Druckmesssignale an die Kontroll- und Auswerteeinrichtung des Analysegeräts auszugeben. Anhand des in den Kammern herrschenden Drucks kann die Kontroll- und Auswerteeinrichtung die aktuell in den Kammern gespeicherte Sauerstoffmenge ermitteln. Die Kontroll- und Auswerteeinrichtung kann vorteilhafterweise dazu ausgestaltet sein, Betriebsphasen des Analysegeräts, die einen erhöhten Sauerstoffbedarf aufweisen, erst dann zu starten, wenn eine ausreichende Menge Sauerstoff in der Gasspeichereinrichtung vorliegt.

Die Kontroll- und Auswerteeinrichtung des Analysegeräts kann dazu ausgestaltet sein, das Analysegerät, insbesondere die Steuerungseinrichtung zur Flussregulierung, die Ventilbaugruppe und/oder das Volumen der Kammern der Gasspeichereinrichtung, zur Durchführung von Messungen zur Bestimmung der Messgröße zu steuern. Zusätzlich kann die Kontroll- und Auswerteeinrichtung des Analysegeräts dazu ausgestaltet sein, das Analysegerät zur Durchführung von Diagnose- oder Wartungsverfahren zu steuern. Sie kann als zentrale elektronische Datenverarbeitungseinrichtung des Analysegeräts ausgestaltet sein. In einer alternativen Ausgestaltung kann die Kontroll- und Auswerteeinrichtung auch aus mehreren voneinander räumlich getrennten, aber miteinander zur Kommunikation verbundenen einzelnen elektronischen Datenverarbeitungseinrichtungen gebildet sein.

Die erwähnte Messeinrichtung des Analysegeräts umfasst mindestens einen spezifischen Detektor zur Erfassung des Gehalts einer oder mehrerer vorgegebener Verbindungen, z.B. $CO_2$ oder $NO_x$, in einem den Aufschlussreaktor über den Gasauslass verlassenden Gasstrom. Beispielsweise kann das Analysegerät als spezifischen Detektor zur Bestimmung eines TOC-Wertes einen Infrarotdetektor umfassen, der dazu ausgestaltet ist, ein von dem $CO_2$-Gehalt des Gasstroms abhängiges Messsignal zu erzeugen. Alternativ oder zusätzlich kann das Analysegerät als spezifischen Detektor zur Bestimmung eines $TN_b$-Wertes einen Chemilumineszenz-Detektor (CLD-Detektor) umfassen. Der spezifische Detektor zur Bestimmung des $TN_b$-Wertes kann auch dazu ausgestaltet sein, einen auf Infrarot-Detektion oder einer elektrochemischen Messung basierendes Messsignal zu erzeugen. Handelt es sich bei dem Analysegerät um einen Elementaranalysator kann die Messeinrichtung einen für das zu bestimmende Element, z.B. Kohlenstoff, Schwefel, Stickstoff, Wasserstoff und/oder Chlor, spezifischen Detektor umfassen. Solche Detektoren sind im Stand der Technik an sich bekannt.

Die Sauerstofferzeugungseinrichtung kann eine oder mehrere einseitig geschlossene sauerstoffpermeable Membranröhren aus einem keramischen Material mit Perowskitstruktur umfassen, welche eine dem Reaktionsraum zugewandte Retentatseite und eine dem Inneren der Röhre zugewandte Permeatseite aufweist bzw. aufweisen. Herrscht an der Permeatseite ein geringerer Sauerstoffpartialdruck als an der Retentatseite, wird oberhalb einer Mindestbetriebstemperatur der Membranröhren Sauerstoff aus auf der Permeatseite zugeführter Luft durch die Membran ins Innere der Membranröhren transportiert. Zur Erzielung der Mindestbetriebstemperatur wird eine Heizvorrichtung des Aufschlusssystems genutzt.

Die Erfindung umfasst auch ein Verfahren zur Bestimmung einer von der Konzentration eines oder mehrerer Inhaltsstoffe einer Probe abhängigen Messgröße, insbesondere mit einem Analysegerät nach einer der voranstehend beschriebenen Ausgestaltungen, mit den Schritten:

- kontinuierliches Erzeugen einer pro Zeiteinheit im Wesentlichen konstanten Menge an Sauerstoff mittels einer Sauerstofferzeugungseinrichtung;

- Transportieren des erzeugten Sauerstoffs in eine Gasspeichereinrichtung, welche mindestens eine zur Aufnahme einer zu speichernden Sauerstoffmenge dienende Kammer mit einem variablen Volumen aufweist, wobei die Kammer über eine in die Kammer mündende Gaszuleitung mit einem Gasauslass der Sauerstofferzeugungseinrichtung verbunden ist, und wobei die Kammer einen Gasauslass aufweist, welcher, insbesondere über eine Steuerungseinrichtung zur Flussregulierung mindestens eines von der Gasspeichereinrichtung zum Aufschlussreaktor strömenden Sauerstoffstroms, mit einem Gaseinlass eines Aufschlussreaktors verbunden ist;

- Entnehmen einer aktuell benötigten Sauerstoffmenge aus der Gasspeichereinrichtung und Transportieren der entnommenen Sauerstoffmenge in den Aufschlussreaktor und/oder zu einem oder mehreren weiteren Verbrauchern;

- Durchführen eines Aufschlusses der Probe im Aufschlussreaktor und Einleiten von aus der Gasspeichereinrichtung entnommenem Sauerstoff in den Aufschlussreaktor;

- Erfassen eines von der Messgröße abhängigen Messwerts in einem aus dem Aufschlussreaktor ausgeleiteten Gasstrom.

Das erfindungsgemäße Verfahren kann weiter den folgenden Schritt umfassen:

- Transportieren des erzeugten Sauerstoffs in die Kammer mittels einer Pumpe, wobei ein Wandungsbereich der Kammer bei Zunahme einer in der Kammer enthaltenen Sauerstoffmenge unter Vergrößerung des Volumens der Kammer gegen eine Rückstellkraft ausgelenkt wird,

so dass in der Kammer ein von einer Förderrate, mit der der erzeugte Sauerstoff transportiert wird, abhängiges Volumen des erzeugten Sauerstoffs unter einem durch die Rückstellkraft bestimmten Druck gespeichert wird.

Die Kammer der Gasspeichereinrichtung kann durch einen auf einer Seite durch einen Boden verschlossenen Zylinder und einen in dem Zylinder axial bezüglich der Zylinderachse beweglichen, den Zylinder auf seiner dem Boden gegenüberliegenden Seite verschließenden Kolben gebildet sein, wobei der erzeugte Sauerstoff, insbesondere mittels einer Pumpe, mit einer Förderrate in die Kammer transportiert wird, so dass in der Kammer ein von der Förderrate abhängiges Volumen des erzeugten Sauerstoffs unter einem durch die Gewichtskraft des Kolbens und den Querschnitt des Zylinders bestimmten Druck gespeichert wird.

Das Entnehmen der aktuell benötigten Sauerstoffmenge kann anhand eines Ausmaßes einer Auslenkung des erwähnten auslenkbaren Wandungsbereichs der Kammer, insbesondere anhand der axialen Position des Kolbens, gesteuert und/oder geregelt werden.

Umfasst die Gasspeichereinrichtung in einer alternativen Ausgestaltung mindestens zwei Kammern, deren Volumen mittels einer Steuerung veränderbar ist, kann das erfindungsgemäße Verfahren alternativ weiter die Schritte umfassen:

- Transportieren des erzeugten Sauerstoffs in eine erste der zwei Kammern unter Vergrößerung des Volumens der Kammer, bis ein vorgegebenes Volumen und ein vorgegebener Regeldruck in der Kammer erreicht sind;
- Nach Erreichen des vorgegebenen Volumens und des vorgegebenen Regeldrucks in der ersten Kammer Entnehmen der aktuell benötigten Sauerstoffmenge aus der ersten Kammer.

In dieser Verfahrensvariante kann während des Entnehmens der aktuell benötigten Sauerstoffmenge aus der ersten Kammer der von der Sauerstofferzeugungseinrichtung erzeugte Sauerstoff in die andere, zweite Kammer der mindestens zwei Kammern transportiert werden. So können die beiden Kammern abwechselnd befüllt bzw. entleert werden.

Das Verfahren kann vollständig automatisch mittels der Kontroll- und Steuereinrichtung des Analysegeräts durchgeführt werden.

Die Erfindung wird im Folgenden anhand der in den Figuren dargestellten Ausführungsbeispiele näher erläutert. Es zeigen:

Fig. 1　eine schematische Darstellung eines Analysegeräts;

Fig. 2　eine schematische Darstellung eines ersten Ausführungsbeispiels einer Gasspeichereinrichtung für ein Analysegerät;

Fig. 3　eine schematische Darstellung eines zweiten Ausführungsbeispiels einer Gasspeichereinrichtung für ein Analysegerät.

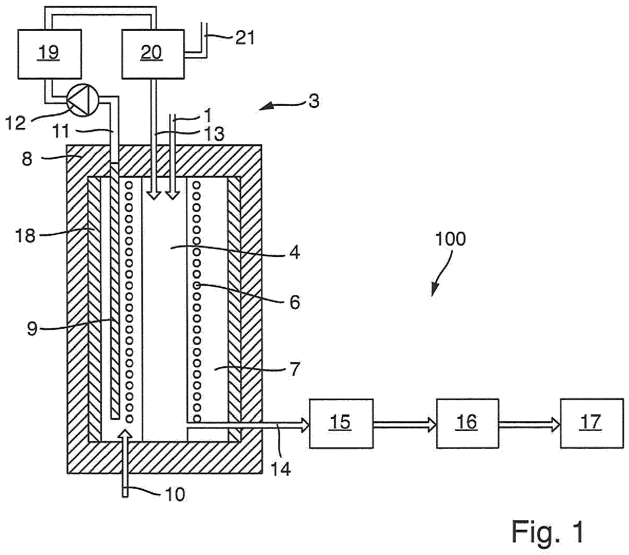

Das schematisch in Fig. 1 dargestellte Analysegerät 100 weist ein Probendosiersystem 1 auf, über das eine Probe einer zu untersuchenden Substanz einem thermischen Aufschlusssystem 3 zuführbar ist. Das Aufschlusssystem 3 umfasst einen rohrförmigen Aufschlussreaktor 4, welcher mittels einer Heizvorrichtung 6 beheizbar ist. Die Heizvorrichtung 6 ist im vorliegenden Beispiel als elektrische Widerstandsheizung ausgestaltet, welche einen spiralförmig um den Aufschlussreaktor verlaufenden Heizleiter umfasst. Das Aufschlusssystem 3 ist in einem Gehäuse 8 untergebracht, in dem auch eine Sauerstofferzeugungseinrichtung mit mehreren sauerstoffpermeablen Membranen 9 (der Übersichtlichkeit halber ist in Fig. 1 nur eine Membran dargestellt) und einem in Fig. 1 nicht näher dargestellten, den Aufschlussreaktor 4 umgebenden Speisegasleitungssystem 7 angeordnet ist. Der Aufschlussreaktor 4, die Heizvorrichtung 6 und das Speisegasleitungssystem 7 sind mittels eines diese umgebenden Isolierrohrs 18 gegenüber dem Gehäuse 8 thermisch isoliert.

Die Membranen 9 sind im vorliegenden Beispiel als aus einem keramischen Material mit Sauerstoffionen-Leitfähigkeit gebildete Röhren ausgestaltet. Geeignete keramische Materialien sind beispielsweise Oxide mit Perowskitstruktur. Perowskite sind ternäre Oxide mit der Gitterstruktur des $ABO_3$-Typs. Oberhalb einer materialabhängigen Mindestbetriebstemperatur, die zwischen 500 und 1000 °C liegen kann, weisen diese Materialien eine simultane elektrische Leitfähigkeit und Sauerstoffionen-Leitfähigkeit auf. Somit ist es möglich, Sauerstoff durch eine gasdichte keramische Membran zu transportieren, wobei eine Abtrennung des Sauerstoffs von anderen Bestandteilen eines der Membran zugeführten Speisegases, das zum Beispiel Luft sein kann, erfolgt. Herrscht auf der Außenseite der Röhren (auch als Retentatseite bezeichnet) ein höherer Sauerstoffpartialdruck als auf der dem Inneren der Röhren zugewandten Innenseite (auch als Permeatseite bezeichnet), so werden bei Temperaturen oberhalb der Mindestbetriebstemperatur der Membran, Sauerstoffmoleküle an der Retentatseite zu negativ geladenen Sauerstoffionen reduziert, Sauerstoffionen von der Retentatseite zur Permeatseite durch die Membran transportiert und an der Permeatseite Sauerstoffionen zu molekularem Sauerstoff oxidiert. Auf diese Weise wird Sauerstoff durch die Membran transportiert und von den auf der Retentatseite verbleibenden übrigen Bestandteilen des Speisegases getrennt. Geeignete Materialien sind beispielsweise perowskitartige Oxide wie $Ba_{1-x}Sr_xCo_{1-y}Fe_yO_{3-\delta}$, speziell z. B. $Ba_{0,5}Sr_{0,5}Co_{0,8}Fe_{0,2}$, oder $La_{1-x}Sr_xCo_{1-y}Fe_y,O_{3-\delta}$, speziell z.B. $La_{0,2}Sr_{0,8}Co_{0,5}Fe_{0,5}O_{3-\delta}$, sowie Nickel/Kobalt-Perowskit-Oxid mit der Zusammensetzung $La_{0,5}Sr_{0,5}Co_{0,8}Ni_{0,2}O_{3-\delta}$.

Die als Röhren ausgestalteten Membranen 9 sind an einem Ende verschlossen. Ihr entgegengesetztes Ende ist über eine Gasleitung 11 mit einer Pumpe 12 verbunden, welche dazu ausgestaltet ist, im Inneren der Röhren einen Unterdruck zu erzeugen, so dass zwischen Retentatseite und Permeatseite das für den Sauerstofftransport erforderliche Sauerstoffpartialdruck-Gefälle entsteht. Als Speisegas wird im hier gezeigten Beispiel Luft aus der Umgebung des Analysegeräts 100 über den Einströmkanal 10 in das Speisegasleitungssystem 7 eingeleitet. Es können auch mehrere Einströmkanäle vorhanden sein, hier ist der Übersichtlichkeit halber nur ein Einströmkanal dargestellt.

Das Analysegerät 100 benötigt und verwendet keine zusätzlichen Heizmittel oder Wärmetauscher zur Heizung der Membranen 9 auf eine Temperatur oberhalb der Mindestbetriebstemperatur. Die zur Heizung des Aufschlussreaktors 4 dienende Heizvorrichtung 6 dient gleichzeitig zur Heizung der Membranen 9.

Mittels der Pumpe 12 wird der auf der Permeatseite der Membranen 9 anfallende gasförmige Sauerstoff in eine Gasspeichereinrichtung 19 geleitet. Über eine Steuerungseinrichtung 20 zur Flussregulierung kann der Gasspeichereinrichtung 19 nach Bedarf eine jeweils aktuell benötigte Sauerstoffmenge entnommen und über den Gaseinlass 13 in den Aufschlussreaktor 4 eingeleitet oder über eine weitere Gasleitung 21 weiteren Sauerstoff-Verbrauchern des Analysegeräts zugeführt werden. Im vorliegenden Beispiel dient der Sauerstoff gleichzeitig als Trägergas und als Oxidationsmittel für die Inhaltsstoffe der in den Aufschlussreaktor über das Probendosiersystem 1 eingebrachten Probe. Die Steuerungseinrichtung 20 ist daher dazu ausgestaltet, kontinuierlich einen vorgegebenen Gasstrom durch den Aufschlussreaktor aufrechtzuerhalten. Die Steuerungseinrichtung 20 umfasst ein Gasleitungs- und Ventilsystem, sowie eine elektronische Kontrollschaltung, die zum Betätigen des Ventilsystems ausgestaltet ist.

Der Aufschluss der Probe wird bei einer Temperatur zwischen 500 und 1000 °C durchgeführt, die mittels der Heizvorrichtung 6 erreicht wird. Der Aufschlussreaktor 4 weist neben dem Gaseinlass 13 auch einen Gasauslass 14 auf, der den Aufschlussreaktor 4 mit einem Trockner 15 verbindet. Der Trockner 15 ist über eine Gasleitung mit einem Detektor 16 verbunden, der dazu ausgestaltet ist, ein von der zu bestimmenden Messgröße abhängiges Messsignal auszugeben. Im vorliegenden Beispiel eines TOC-Analysegeräts kann es sich bei dem Detektor 16 um einen Infrarotdetektor handeln, der dazu ausgestaltet ist, ein vom $CO_2$-Gehalt des aus dem Gasauslass 14 über den Trockner 15 dem Detektor 16 zugeleiteten Gasstrom abhängiges Messsignal zu erzeugen. Der Detektor 16 ist mit einer Kontroll- und Auswerteeinrichtung 17 verbunden, die dazu ausgestaltet ist, das Messsignal des Detektors 16 zu erfassen und anhand des Messsignals einen Messwert der Messgröße, hier des TOC-Werts der Probe, zu ermitteln. Bei der Kontroll- und Auswerteeinrichtung 17 kann es sich beispielsweise um eine elektronische Datenverarbeitungseinrichtung, insbesondere einen PC, handeln, der ein der Bestimmung der Messgröße dienendes Auswerteprogramm umfasst und ausführen kann.

Die Kontroll- und Auswerteeinrichtung 17 dient neben der Auswertung der Messsignale auch zur Steuerung des Analysegeräts 100. Sie ist dazu ausgestaltet, die Heizeinrichtung 6, die Pumpe 12, ggfs. vorhandene Antriebe der Gasspeichereinrichtung 19 und die Steuerungseinrichtung 20 zur Flussregulierung zu steuern. Die Kontroll- und Auswerteeinrichtung 17 kann in Form einer einzigen, zentralen Datenverarbeitungseinheit ausgestaltet sein. Alternativ kann sie mehrere einzelne, gegebenenfalls voneinander räumlich getrennte Datenverarbeitungseinheiten umfassen, die jeweils einzelne Komponenten des Analysegeräts bzw. der Gasspeichereinrichtung steuern und die miteinander zur Kommunikation verbunden sind.

Die Gesamtfläche der Membranröhren 9 zur Sauerstofferzeugung ist dazu ausgelegt kontinuierlich einen Sauerstoffstrom zwischen 50 ml bis 5 l/min zur Verfügung zu stellen. Das Analysegerät 100 ist jedoch so ausgelegt, dass eine in bestimmten Betriebsphasen von dem Aufschlussreaktor 4 und den weiteren Sauerstoff-Verbrauchern insgesamt benötigte Maximalmenge an Sauerstoff die während dieser Betriebsphasen durch die Sauerstofferzeugungseinrichtung erzeugte Sauerstoffmenge übersteigen kann. Die Gasspeichereinrichtung 19 ist dazu ausgestaltet, in Betriebsphasen, in denen der Aufschlussreaktor 4 und die weiteren Sauerstoff-Verbraucher zusammen weniger als die während dieser Betriebsphasen von der Sauerstofferzeugungseinrichtung erzeugte Sauerstoffmenge benötigen, den nicht benötigten Überschuss an Sauerstoff vorübergehend zu speichern. Der gespeicherte Sauerstoff steht in Betriebsphasen zur Verfügung, in denen die insgesamt benötigte Sauerstoffmenge die während dieser Betriebsphasen von der Sauerstofferzeugungsvorrichtung erzeugte Sauerstoffmenge übersteigt. Dabei ist die Gasspeichereinrichtung so ausgelegt, dass jederzeit die für die aktuelle Betriebsphase benötigte Sauerstoffmenge und ein zusätzlicher Sicherheitsüberschuss, der beispielsweise etwa 10% betragen kann, zur Verfügung stehen.

Es ist auch möglich, die Kontroll- und Auswerteeinrichtung 17 dazu auszugestalten, die Durchführung von Messzyklen des Analysegeräts 100 so zu steuern, dass vor einer Betriebsphase mit erhöhtem, d.h. die kontinuierlich von der Sauerstofferzeugungsvorrichtung zur Verfügung gestellte Sauerstoffmenge übersteigenden, Sauerstoffbedarf zunächst geprüft wird, ob in der Gasspeichereinrichtung eine ausreichende Menge an zusätzlichem Sauerstoff gespeichert ist und den Beginn der Betriebsphase so lange zu verzögern, bis eine ausreichende Menge an Sauerstoff in der Gasspeichereinrichtung vorliegt.

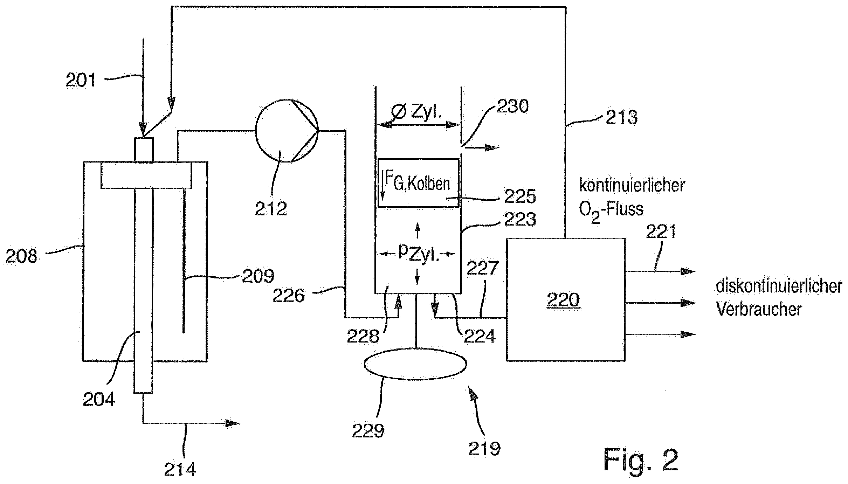

In Fig. 2 ist schematisch ein erstes Ausführungsbeispiel für eine Gasspeichereinrichtung 219 eines Analysegeräts dargestellt. Das Analysegerät ist im Wesentlichen ausgestaltet, wie das anhand von Fig. 1 beschriebene Analysegerät 100. Insbesondere umfasst es einen in einem Gehäuse 208 untergebrachten, beheizbaren Aufschlussreaktor 204, dem über eine Probenzuführung 201 eine zu analysierende Probe zuführbar ist. Weiter umfasst das Analysegerät eine Sauerstofferzeugungseinrichtung mit Membranrohren 209, die zur Erzeugung von Sauerstoff aus der Umgebungsluft dienen. Die innere Permeatseite der Membranrohre 209 ist über eine Vakuumpumpe 212 mit der Gasspeichereinrichtung 219 verbunden.

Die Gasspeichereinrichtung 219 umfasst einen Zylinder 223, der auf einer Seite durch einen Boden 224 verschlossen ist. In dem Zylinder ist ein Kolben 225 axial bezüglich der Zylinderachse beweglich geführt, der im hier gezeigten Ausführungsbeispiel den Zylinder gasdicht verschließt. Die rohrförmige Zylinderwandung, der Boden 224 und der Kolben 225 schließen eine Kammer 228 ein, in die eine mit der Sauerstofferzeugungseinrichtung über die Vakuumpumpe 212 verbundene Gaszuleitung 226 und eine mit der Steuerungseinrichtung 220 zur Flusskontrolle verbundene Gasableitung 227 münden. Die Gasspeichereinrichtung 219 kann weiter einen Drucksensor 229 umfassen, welcher dazu ausgestaltet ist, den in der Kammer 228 herrschenden Gasdruck zu erfassen. In die Zylinderwandung mündet im hier beschriebenen Beispiel in einem oberen Bereich ein weiterer Gasauslass 230 ein.

Die Steuerungseinrichtung 220 ist im Wesentlichen ausgestaltet wie die Steuerungseinrichtung 20 des in Fig. 1 dargestellten Analysegeräts 100. Sie ist über ein Gasleitungssystem 213 mit dem Aufschlussreaktor 204 und weiteren Verbrauchern 221 verbunden. Die Steuerungseinrichtung 220 umfasst eine elektronische Kontrollschaltung und eine mittels der Kontrollschaltung betätigbare Ventileinrichtung. Durch Steuerung der Ventileinrichtung kann die Steuerungseinrichtung 220 einen von der Kammer 228 kommenden Sauerstoffstrom dem Aufschlussreaktor 204 über die Zuleitung 213 und/oder weiteren Verbrauchern über die Zuleitungen 221 zuführen.

Die Kammer 228 der Gasspeichereinrichtung 219 weist aufgrund der axialen Beweglichkeit des Kolbens 225 ein variables Volumen auf. Dieses Speichervolumen ist so ausgelegt, dass sie etwa die Fördermenge von in 3 bis 30 min von der Sauerstofferzeugungseinrichtung erzeugten Sauerstoffs aufnehmen kann. Der Kolben 225 wird von dem mittels der Pumpe 212 in die Kammer eingeleiteten Sauerstoff gegen seine als Rückstellkraft wirkende Gewichtskraft verdrängt, wobei eine konstante Druckdifferenz zur umgebenden Atmosphäre aufgebaut wird. Die Druckdifferenz ist durch die Gewichtskraft und die Fläche des Kolbens 225 bestimmt. Unter Vernachlässigung der bei der axialen Bewegung des Kolbens 225 auftretenden Reibung beträgt der Druck $p_{zyl}$ in der Kammer 228:

$$p_{zyl} = \frac{4}{\pi} \cdot \frac{F_{G,Kolben}}{\emptyset_{zyl}^2},$$

wobei $F_{G,Kolben}$ die Gewichtskraft des Kolbens und $\emptyset_{zyl}$ den Zyinderdurchmesser bezeichnet.

Dieser konstante Überdruck dient zur Flussregulierung des Hauptgasstroms zum Aufschlussreaktor 204 und der Hilfsgasströme zu den weiteren Verbrauchern. Damit ist der Einsatz weiterer Druckregler und Stellventile unnötig. Es reicht aus, dass die Pumpe 212 zusätzlich zum systembedingten, dem Sauerstofftransport dienenden Unterdruck im Fördersystem noch einen geringen Überdruck von beispielsweise 20 bis 100 mbar für das Speichersystem erzeugt. Nimmt die in der Kammer 228 vorhandene Sauerstoffmenge so stark zu, dass der Kolben 225 über das Niveau des Gasauslass 230 angehoben wird, entweicht Sauerstoff in die Umgebung, was zu einer Abwärtsbewegung des Kolbens 225 führt. Auf diese Weise ist sichergestellt, dass das Volumen der Kammer 228, und damit die gespeicherte Sauerstoffmenge, einen durch die Position des Gasauslass 230 vorgegebenen Maximalwert nicht überschreitet.

Das durch die Bewegung des Kolbens 225 variable Speichervolumen gewährleistet ein möglichst geringes Totvolumen der Gasspeichereinrichtung 219.

Ein Verfahren zum Speichern von Sauerstoff in der Gasspeichereinrichtung 219 und ein Verfahren zum Betreiben der Gasspeichereinrichtung 219, das den Aufschlussreaktor 204 und weitere Verbraucher jeweils bedarfsgerecht mit Sauerstoff versorgt, wird im Folgenden beschrieben:

Das Analysegerät führt gesteuert durch eine Kontroll- und Auswerteeinrichtung, die in gleicher Weise ausgestaltet sein kann wie die anhand von Fig. 1 beschriebene Kontroll- und Auswerteeinrichtung 17 des Analysegeräts 100, in regelmäßigen Abständen oder ereignisgesteuert Messzyklen zur Bestimmung der Messgröße oder Wartezeiten durch. Die Messzyklen können in einzelne Betriebsphasen unterteilt werden. In jeder Betriebsphase leitet die Steuerungseinrichtung 220 dem Aufschlussreaktor 204 und nach Bedarf einem oder mehreren weiteren Verbrauchern die in dieser Betriebsphase benötigte Sauerstoffmenge zu. Dabei wird der Sauerstoff aus der Kammer 228 entnommen. Die Steuerungseinrichtung kann dazu von der Kontroll- und Auswerteeinrichtung, mit der sie zur Kommunikation verbunden ist, entsprechende Steuerbefehle erhalten. Alternativ kann die weiter oben erwähnte, die Ventileinrichtung der Steuerungseinrichtung 220 betätigende Kontrollschaltung der Steuerungseinrichtung 220 Bestandteil der Kontroll- und Auswerteeinrichtung des Analysegeräts sein.

Um sicherzustellen, dass zu Beginn eines Abarbeitungszyklus bzw. einer Betriebsphase ein ausreichender Gasvorrat für die Abarbeitung einer anstehenden Sequenz von Betriebsphasen des Analysegeräts vorhanden ist, kann die axiale Position des Kolbens 225 mittels einer geeigneten Sensorik überwacht werden. Die entsprechenden Sensorsignale können von der Kontroll- und Auswerteeinrichtung des Analysegeräts verarbeitet werden. Wird dabei festgestellt, dass der aktuell vorhandene Gasvorrat nicht ausreicht, kann durch Pausieren des Ablaufs der Vorrat aufgefüllt werden. Vorzugsweise erfolgt das Pausieren des Ablaufs nur zu einem Zeitpunkt, an dem ein solches Pausieren kein von dem Analysegerät durchgeführtes Verfahren gefährdet.

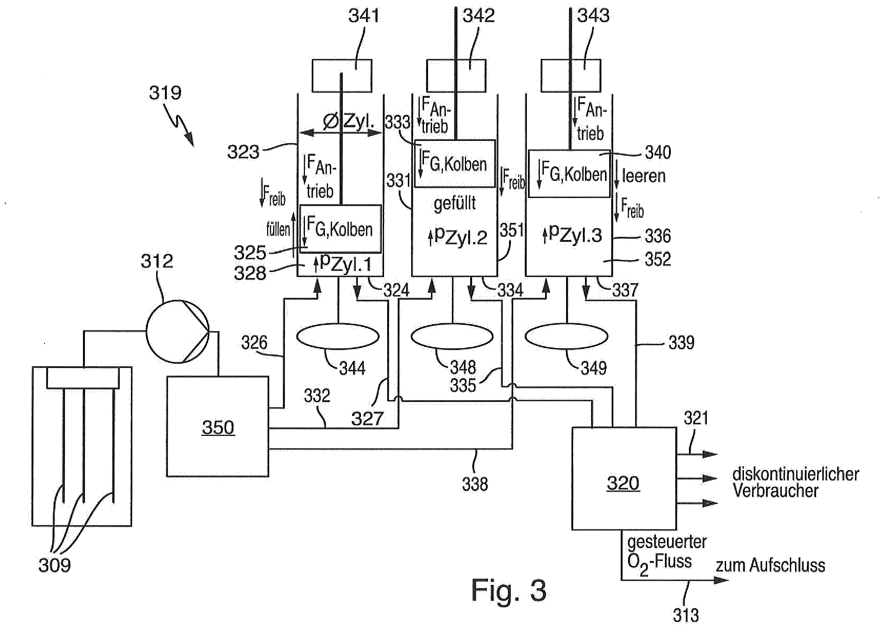

In Fig. 3 ist ein weiteres Ausführungsbeispiel einer Gasspeichereinrichtung 319 dargestellt. Diese kann in einem Analysegerät verwendet werden, das neben einem Aufschlussreaktor weitere Sauerstoff-Verbraucher aufweist, und das eine Sauerstofferzeugungseinrichtung umfasst, die dazu ausgestaltet ist, Sauerstoff aus der Umgebungsluft zu erzeugen. Beispielsweise kommt eine Verwendung der Gasspeichereinrichtung 319 in dem anhand von Fig. 1 beschriebenen Analysegerät in Frage.

Die Sauerstofferzeugungseinrichtung weist mehrere Membranröhren 309 auf, die nach dem weiter oben in Zusammenhang mit Fig. 1 beschriebenen Prinzip kontinuierlich hochreinen Sauerstoff erzeugen. Die Permeatseite der Membranröhren ist über eine Vakuumpumpe 312 mit einem Gasleitungssystem und einer Ventilbaugruppe 350 verbunden, die dazu dienen, den von der Sauerstofferzeugungseinrichtung erzeugten Sauerstoff je nach Bedarf wahlweise einer von drei Kammern 328, 351, 352 der Gasspeichereinrichtung 319 zuzuleiten, die jeweils ein variables Volumen aufweisen.

Die Gasspeichereinrichtung 319 umfasst im hier gezeigten Beispiel drei Zylinder 323, 331, 336, die jeweils auf einer Seite durch einen Boden 324, 334, 337 verschlossen sind, und in denen jeweils ein Kolben 325, 333, 340 axial beweglich geführt ist. Die Kammern 328, 351, 352 sind jeweils durch die rohrförmige Zylinderwandung, den Boden 324, 334, 337 und den die Kammern 328, 351, 352 gasdicht verschließenden Kolben 325, 333, 340 gebildet. In die Kammern münden jeweils eine Zuleitung 326, 332, 338 und eine Ableitung 327, 335, 339 ein. Die Zuleitungen 326, 332, 338 sind über die Ventilbaugruppe 350 mit der Sauerstofferzeugungseinrichtung verbunden, so dass mittels der Ventilbaugruppe der von der Sauerstofferzeugungseinrichtung erzeugte Sauerstoff wahlweise in eine der Kammern 328, 351, 352 geleitet werden kann. Die Ableitungen 327, 335, 339 sind mit einer Steuerungseinrichtung 320 zur Flussregulierung verbunden, die dazu ausgestaltet ist, aus den Kammern 328, 351, 352 entnommenen Sauerstoff über eine Gasleitung 313 einem Aufschlussreaktor des Analysegeräts und/oder über die Gasleitungen 321 einer Vielzahl von diskontinuierlichen Verbrauchern zuzuleiten.

Die axiale Bewegung der Kolben 325, 333, 340 wird mittels der Antriebe 341, 342, 343 bewirkt. Der in den Kammern 328, 351, 352 herrschende Druck wird jeweils mittels eines Drucksensors 344, 348, 349 überwacht. Zum Befüllen einer Kammer 328, 351, 352 mit Sauerstoff wird der jeweilige Kolben 325, 333, 340 unter Vergrößerung des Kammervolumens zurückgezogen, zum Entleeren der Kammer 328, 351, 352 wird der Kolben 325, 333, 340 entsprechend nach unten in Richtung des Bodens 324, 334, 337 unter Verkleinerung des Kammervolumens bewegt.

Die Steuerung der Ventilbaugruppe 350, der Steuereinheit 320 zur Flussregulierung und der Antriebe 341, 342, 343 kann mittels einer elektronischen Datenverarbeitungseinrichtung erfolgen. Dabei kann die Gasspeichereinrichtung 319 eine zu diesem Zweck ausgestaltete, separate Datenverarbeitungseinrichtung besitzen. Diese kann zur Kommunikation mit einer Kontroll- und Steuereinrichtung des Analysegeräts, in dem die Gasspeichereinrichtung 319 eingesetzt wird, verbunden sein. Alternativ kann die Steuereinheit 320 zur Flussregulierung zusätzlich zur Steuerung der Ventilbaugruppe 350 und der Antriebe 341, 342, 343 ausgestaltet sein. Es ist auch möglich, dass eine zentrale Kontroll- und Auswerteeinrichtung des Analysegeräts, in dem die Gasspeichereinrichtung 319 eingesetzt wird, die Steuerung der Ventilbaugruppe 350, der Steuereinheit 320 zur Flussregulierung und der Antriebe 341, 342, 343 übernimmt.

Ein Verfahren zum Speichern von Sauerstoff in der Gasspeichereinrichtung 319 und ein Verfahren zum Betreiben der Gasspeichereinrichtung 319, das den Aufschlussreaktor und weitere Verbraucher jeweils bedarfsgerecht mit Sauerstoff versorgt, wird im Folgenden beschrieben:

In einem ersten Arbeitszyklus (Füllen) wird ein erster der Zylinder - im in Fig. 3 dargestellten Beispiel handelt es sich dabei um den Zylinder 323 - vom Sauerstofferzeugungssystem gespeist und bis zum Erreichen einer bestimmten Füllmenge mit Sauerstoff gefüllt. Die bestimmte Füllmenge ist durch eine vorgegebene axiale Position des Kolbens 325 und einen vorgegebenen, in der Kammer 328 herrschenden Regeldruck bestimmt. Ist die bestimmte Füllmenge erreicht, wird mittels der Ventilbaugruppe 350 die Zuleitung 326 verschlossen, um den Sauerstofftransport in die Kammer 328 zu beenden. Die Ventilbaugruppe 350 kann den weiter kontinuierlich von der Sauerstoffeinrichtung erzeugten Sauerstoffstrom sequentiell den weiteren als Speichervolumina dienenden Kammern der Gasspeichereinrichtung 319 zuleiten. Im hier gezeigten Beispiel ist der zweite Zylinder 331 gerade vollständig mit der vorgegebenen maximalen Sauerstoff-Füllmenge befüllt, während der erste Zylinder 323 befüllt wird. Eine die Ventilbaugruppe 350 und die Antriebe 341, 342, 343 steuernde Datenverarbeitungseinrichtung kann das Füllen der Zylinder 323, 331, 336 vollständig automatisiert basierend auf den von den Drucksensoren 344, 348, 349 gelieferten Messwerten und unter Berücksichtigung der axialen Position der Kolben 325, 333, 340 oder eines von den Kolben 325, 333, 340 zurückgelegten Wegs steuern. Wie bereits erwähnt, kann diese Steuerungsfunktion auch von der Kontroll- und Auswerteeinrichtung des Analysegeräts durchgeführt werden. Zur Erfassung eines von den Kolben 325, 333, 340 zurückgelegten Wegs können jeweils Wegsensoren vorgesehen sein (in Fig. 3 nicht gezeigt), deren Messwerte die Datenverarbeitungseinrichtung erfasst und verarbeitet. Alternativ kann die Datenverarbeitungseinrichtung den von dem Kolben 325, 333, 340 zurückgelegten Weg anhand der Bewegung der Antriebe 341, 342, 343 ermitteln.

In einem zweiten Arbeitszyklus (Dosieren) wird aus einer befüllten Kammer Sauerstoff entnommen und nach aktuellem Bedarf dem Aufschlussreaktor des Analysegeräts und/oder einem oder mehreren weiteren Sauerstoffverbrauchern zugeleitet. Im in Fig. 3 dargestellten Beispiel wird der dritte Zylinder 336 gerade im zweiten Arbeitszyklus betrieben. Hierzu wird mittels der Steuereinheit 320 zur Flussregulierung ein Gasweg bzw. mehrere Gaswege zwischen der Kammer 352 und den mit Sauerstoff zu versorgenden Verbrauchern bzw. dem Aufschlussreaktor freigegeben. Mittels des Antriebs 343 wird der Kolben 340 in Richtung des Bodens 337 der Kammer 352 bewegt und so das Volumen der Kammer 352 verringert. Hierdurch wird eine geregelte Kraft auf den in der Kammer 352 aufgenommen Sauerstoff ausgeübt, welche einen von der Kraft abhängigen Überdruck in der Kammer erzeugt, der dem Transport einer gewünschten Menge Sauerstoff zu dem Aufschlussreaktor bzw. den weiteren Verbrauchern dient. Die Steuerung des Antriebs 343 und der Steuereinheit 320 kann hierbei mittels der erwähnten elektronischen Datenverarbeitungseinrichtung oder durch die Kontroll- und Auswerteeinrichtung des Analysegeräts erfolgen, basierend auf von dem Drucksensor 349 gelieferten Messwerten, dem von dem Kolben 340 während des Dosierens zurückgelegten Weg und einer von der Kontroll- und Auswerteeinrichtung vorgegebenen, für die aktuelle Betriebsphase des Analysegeräts benötigten Sauerstoffmenge.

So wird es ermöglicht, dem Analysegerät zur Durchführung eines Analyseverfahrensschritts eine gesteuerte Menge an Sauerstoff zu Verfügung zu stellen. Diese kann von einer oder mehreren Teilmengen der in den einzelnen Kammern 328 351, 352 enthaltenen Sauerstoffmenge bis zum gesamten Speichervolumen der Gasspeichereinrichtung 319 reichen. Eine Kopplung mehrerer gefüllter Einzelspeichervolumina für eine Summendosierung ist ebenso möglich, wie eine zeitlich synchronisierte Taktung von Füll und Dosier-Arbeitszyklen der einzelnen als Speichereinheiten dienenden Zylinder.

**Patentansprüche**

1. Analysegerät (100) zur Bestimmung einer von der Konzentration eines oder mehrerer Inhaltsstoffe einer Probe abhängigen Messgröße, umfassend:

    - einen Aufschlussreaktor (4, 204) mit einem Gaseinlass (13) und einem Gasauslass (14);
    - eine Messeinrichtung, die mit dem Gasauslass (14) des Aufschlussreaktors (4) verbunden ist;
    - eine Kontroll- und Auswerteeinrichtung (17), welche dazu ausgestaltet ist, von der Messeinrichtung registrierte, von der Messgröße abhängige Messwerte zu empfangen und zu verarbeiten;
    - eine Sauerstofferzeugungseinrichtung; und
    - ein Gasleitungssystem, welches die Sauerstofferzeugungseinrichtung mit dem Gaseinlass (13) des Aufschlussreaktors (4, 204) verbindet, um dem Aufschlussreaktor (4, 204) von der Sauerstofferzeugungseinrichtung erzeugten Sauerstoff zuzuführen,

    **dadurch gekennzeichnet, dass** das Gasleitungssystem eine Gasspeichereinrichtung (19, 219, 319) umfasst, welche mindestens eine zur Aufnahme einer zu speichernden Sauerstoffmenge dienende Kammer (228, 328, 351, 352) mit einem variablen Volumen aufweist.

2. Analysegerät (100) nach Anspruch 1, wobei das Gasleitungssystem eine Steuerungseinrichtung (20, 220, 320) zur Flussregulierung mindestens eines durch das Gasleitungssystem von der Gasspeichereinrichtung (19, 219, 319) zum Aufschlussreaktor (4) strömenden Sauerstoffstroms umfasst.

3. Analysegerät (100) nach Anspruch 1 oder 2, wobei in die Kammer (228, 328, 351, 352) der Gasspeichereinrichtung eine Gaszuleitung (226) mündet, welche, insbesondere über eine Pumpe (212, 312), mit einem Gasauslass der Sauerstofferzeugungseinrichtung verbunden ist, und wobei die Kammer (228, 328, 351, 352) einen Gasauslass aufweist, welcher, insbesondere über die Steuerungseinrichtung (20, 220, 320), mit dem Gaseinlass des Aufschlussreaktors (4, 204) verbunden ist.

4. Analysegerät (100) nach Anspruch 2 oder 3, wobei das Gasleitungssystem die Sauerstofferzeugungseinrichtung über die Gasspeichereinrichtung (219, 319) zusätzlich mit einem oder mehreren weiteren Verbrauchern verbindet, um diesen von der Sauerstofferzeugungseinrichtung erzeugten Sauerstoff zuzuführen und

wobei die Steuerungseinrichtung (220, 320) zusätzlich dazu ausgestaltet ist, einen Durchfluss von Sauerstoff durch das Gasleitungssystem von der Gasspeichereinrichtung (219, 319) zu dem einen oder den mehreren weiteren Verbrauchern zu steuern.

5. Analysegerät (100) nach Anspruch 4,
wobei der Gasauslass der mindestens einen Kammer (228, 328, 351, 352) der Gasspeichereinrichtung (219, 319) über die Steuerungseinrichtung (220, 320) mit dem einen weiteren Verbraucher oder mit einem oder mehreren, insbesondere mit jedem, der weiteren Verbraucher verbunden ist.

6. Analysegerät (100) nach einem der Ansprüche 1 bis 5,
wobei in die Kammer (228) eine Gaszuleitung (226) mündet, welche mit einem Gasauslass der Sauerstofferzeugungseinrichtung verbunden ist, und wobei die Kammer (228) einen Wandungsbereich umfasst, welcher dazu ausgestaltet ist, bei Zunahme einer in der Kammer enthaltenen Sauerstoffmenge unter Vergrößerung des Volumens der Kammer (228) gegen eine Rückstellkraft ausgelenkt zu werden.

7. Analysegerät (100) nach einem der Ansprüche 1 bis 6,
wobei die Kammer (228) der Gasspeichereinrichtung (219) durch einen auf einer Seite durch einen Boden (224) verschlossenen Zylinder (223) und einen in dem Zylinder (223) axial bezüglich der Zylinderachse beweglich geführten, den Zylinder (223) auf seiner dem Boden (224) gegenüberliegenden Seite gasdicht verschließenden Kolben (225) gebildet ist, und wobei die axiale Position des Kolbens (225) von der in der Kammer (228) enthaltenen Sauerstoffmenge abhängt.

8. Analysegerät (100) nach Anspruch 7,
wobei das Gasleitungssystem Mittel zur Überwachung der axialen Position des Kolbens (225) umfasst.

9. Analysegerät (100) nach einem der Ansprüche 1 bis 5,
wobei die Gasspeichereinrichtung (319) mindestens zwei Kammern (328, 351, 352) aufweist, deren Volumen mittels einer Steuerung veränderbar ist, wobei in jede der Kammern (328, 351, 352) eine Gaszuleitung mündet, welche mit dem Gasauslass der Sauerstofferzeugungseinrichtung verbindbar ist, und wobei jede der Kammern (328, 351, 352) einen Gasauslass aufweist, welcher, insbesondere über die Steuerungseinrichtung (320) zur Flussregulierung, mit dem Gaseinlass des Aufschlussreaktors verbunden ist.

10. Analysegerät (100) nach Anspruch 9,
wobei jede der Gaszuleitungen über eine Ventilbaugruppe (350) mit dem Gasauslass der Sauerstofferzeugungseinrichtung verbunden ist, und wobei die Ventilbaugruppe (350) dazu ausgestaltet ist, vom Gasauslass der Sauerstofferzeugungseinrichtung zu den Kammern (328, 351, 352) führende Gasleitungen wahlweise, insbesondere abwechselnd oder nach einem von der Steuerung vorgegebenen Ablauf, zu sperren oder freizugeben.

11. Analysegerät (100) nach Anspruch 9 oder 10,
wobei die mindestens zwei Kammern (328, 351, 352) jeweils durch einen auf einer Seite durch einen Boden (324, 334, 337) verschlossenen Zylinder (323, 331, 336) und einen in dem Zylinder (323, 331, 336) axial bezüglich der Zylinderachse beweglichen Kolben (325, 333, 340) gebildet sind, welcher den Zylinder gasdicht verschließt,
und wobei jeder Kolben (325, 333, 340) mit einem eine axiale Bewegung des Kolbens (325, 333, 340) bewirkenden, insbesondere von der Steuerung betätigbaren, Antrieb (341, 342, 343) verbunden ist.

12. Verfahren zur Bestimmung einer von der Konzentration eines oder mehrerer Inhaltsstoffe einer Probe abhängigen Messgröße, insbesondere mit einem Analysegerät (100) nach einem der Ansprüche 1 bis 11, umfassend:

    - kontinuierliches Erzeugen einer pro Zeiteinheit im Wesentlichen konstanten Menge an Sauerstoff mittels einer Sauerstofferzeugungseinrichtung;
    - Transportieren des erzeugten Sauerstoffs in eine Gasspeichereinrichtung (19, 219, 319), welche mindestens eine zur Aufnahme einer zu speichernden Sauerstoffmenge dienende Kammer (228, 328, 351, 352) mit einem variablen Volumen aufweist, wobei die Kammer (228, 328, 351, 352) über eine in die Kammer (228, 328, 351, 352) mündende Gaszuleitung (226) mit einem Gasauslass der Sauerstofferzeugungseinrichtung verbunden ist, und wobei die Kammer (228, 328, 351, 352) einen Gasauslass aufweist, welcher, insbesondere über eine Steuerungseinrichtung (20, 220, 320) zur Flussregulierung mindestens eines von der Gasspeichereinrichtung (19, 219, 319) zum Aufschlussreaktor (4, 204) strömenden Sauerstoffstroms, mit einem Gaseinlass eines Aufschlussreaktors (4, 204) verbunden ist;
    - Entnehmen einer aktuell benötigten Sauerstoffmenge aus der Gasspeichereinrichtung (19, 219, 319) und Transportieren der entnommenen Sauerstoffmenge in den Aufschlussreaktor (4, 204) und/oder an einen oder mehrere weitere Verbraucher;

- Durchführen eines Aufschlusses der Probe im Aufschlussreaktor (4, 204) und Einleiten von aus der Gasspeichereinrichtung entnommenem Sauerstoff in den Aufschlussreaktor (4, 204); und

- Erfassen eines von der Messgröße abhängigen Messwerts in einem aus dem Aufschlussreaktor (4, 204) ausgeleiteten Gasstrom.

13. Verfahren nach Anspruch 12,
weiter umfassend:

- Transportieren des erzeugten Sauerstoffs in die Kammer (228) mittels einer Pumpe (212), wobei ein Wandungsbereich der Kammer (228) bei Zunahme einer in der Kammer (228) enthaltenen Sauerstoffmenge unter Vergrößerung des Volumens der Kammer (228) gegen eine Rückstellkraft ausgelenkt wird,

so dass in der Kammer (228) ein von einer Förderrate, mit der der erzeugte Sauerstoff transportiert wird, abhängiges Volumen des erzeugten Sauerstoffs unter einem durch die Rückstellkraft bestimmten Druck gespeichert wird.

14. Verfahren nach Anspruch 12,
wobei die Gasspeichereinrichtung (319) mindestens zwei Kammern (328, 351, 352) umfasst, deren Volumen mittels einer Steuerung veränderbar ist, weiter umfassend die Schritte:

- Transportieren des erzeugten Sauerstoffs in eine erste der zwei Kammern (328, 351, 352) unter Vergrößerung des Volumens der Kammer (328, 351, 352), bis ein vorgegebenes Volumen und ein vorgegebener Regeldruck in der Kammer (328, 351, 352) erreicht sind; und
- Nach Erreichen des vorgegebenen Volumens und des vorgegebenen Regeldrucks in der ersten Kammer Entnehmen der aktuell benötigten Sauerstoffmenge aus der ersten Kammer.

15. Verfahren nach Anspruch 14,
wobei während des Entnehmens der aktuell benötigten Sauerstoffmenge aus der ersten Kammer der von der Sauerstofferzeugungseinrichtung erzeugte Sauerstoff in die andere, zweite Kammer der mindestens zwei Kammern (328, 351, 352) transportiert wird.

**Claims**

1. Analyzer (100) designed to determine a measured variable that depends on the concentration of one or more ingredients in a sample, said device comprising:

- a digestion reactor (4, 204) with a gas inlet (13) and a gas outlet (14);
- a measuring device, which is connected to the gas outlet (14) of the digestion reactor (4);
- a control and evaluation unit (17), which is designed to receive and process measured values that are registered by the measuring device and depend on the measured variable;
- an oxygen generation device; and
- a gas pipe system, which connects the oxygen generation device to the gas inlet (13) of the digestion reactor (4, 204) for the purpose of supplying the digestion reactor (4, 204) with oxygen generated by the oxygen generation device,

**characterized in that** the gas pipe system comprises a gas storage unit (19, 219, 319), wherein said unit has at least a chamber (228, 328, 351, 352) with a variable volume that serves to hold a quantity of oxygen to be stored.

2. Analyzer (100) as claimed in Claim 1,
wherein the gas pipe system comprises a control unit (20, 220, 320) for the flow regulation of at least an oxygen current flowing through the gas pipe system from the gas storage unit (19, 219, 319) to the digestion reactor (4).

3. Analyzer (100) as claimed in Claim 1 or 2,
wherein a gas supply pipe (226) enters into the chamber (228, 328, 351, 352) of the gas storage unit, said gas supply pipe being connected, particularly via a pump (212, 312), to a gas outlet of the oxygen generation device, and wherein the chamber (228, 328, 351, 352) has a gas outlet which is connected, particularly via the control unit (20, 220, 320), to the gas inlet of the digestion reactor (4, 204).

4. Analyzer (100) as claimed in Claim 2 or 3,
wherein the gas pipe system further additionally connects the oxygen generation device to one or more other consumers via the gas storage unit (219, 319) to supply said consumers with oxygen generated by the oxygen generation device, and
wherein the control unit (220, 320) is further designed to control an oxygen flow through the gas pipe system from the gas storage unit (219, 319) to one or more consumers.

5. Analyzer (100) as claimed in Claim 4,
wherein the gas outlet of the at least one chamber (228, 328, 351, 352) of the gas storage unit (219, 319) is connected via the control unit (220, 320) to the one other consumer or to one or more, particularly to all, of the other consumers.

6. Analyzer (100) as claimed in one of the Claims 1 to 5, wherein a gas supply pipe (226) enters into the chamber (228) and is connected to a gas outlet of the oxygen generation device, and wherein the chamber (228) comprises a wall area which is designed to be deflected against a reset force when a quantity of oxygen contained in the chamber increases by also increasing the volume of the chamber (228).

7. Analyzer (100) as claimed in one of the Claims 1 to 6, wherein the chamber (228) of the gas storage unit (219) is formed by a cylinder (223) sealed on one side by a floor (224) and a piston (225) guided in the cylinder (223) in a manner in which it is axially mobile in relation to the axis of the cylinder and closes the cylinder (223) in a gas-tight manner on its side opposite the floor (224), and
wherein the axial position of the piston (225) depends on the quantity of oxygen contained in the chamber (228).

8. Analyzer (100) as claimed in Claim 7, wherein the gas pipe system comprises means to monitor the axial position of the piston (225).

9. Analyzer (100) as claimed in one of the Claims 1 to 5, wherein the gas storage unit (319) has at least two chambers (328, 351, 352) whose volume can be modified by means of a controller, wherein a gas supply pipe enters into each of the chambers (328, 351, 352), wherein said pipe can be connected to the gas outlet of the oxygen generation device, and wherein each of the chambers (328, 351, 352) has a gas outlet which is connected to the gas inlet of the digestion reactor particularly via the control unit (320) that is used to regulate the flow.

10. Analyzer (100) as claimed in Claim 9, wherein each of the gas supply pipes is connected to the gas outlet of the oxygen generation device via a valve assembly (350), and
wherein the valve assembly (350) is designed to selectively block or release the gas pipes leading from the gas outlet of the oxygen generation device to the chambers (328, 351, 352), particularly alternatively or according to a sequence predefined by the controller.

11. Analyzer (100) as claimed in Claim 9 or 10, wherein the at least two chambers (328, 351, 352) are each formed by a cylinder (323, 331, 336) sealed on a side by a floor (324, 334, 337) and by a piston (325, 333, 340) which is axially mobile in the cylinder (323, 331, 336) in relation to the axis of the cylinder and which seals the cylinder in a gas-tight manner, and wherein each piston (325, 333, 340) is connected to a drive (341, 342, 343) which produces an axial movement of the piston (325, 333, 340) and can particularly be actuated by the controller.

12. Procedure designed to determine a measured variable that depends on the concentration of one or more ingredients of a sample, particularly with an analyzer (100) as claimed in one of the Claims 1 to 11, said procedure comprising the following steps:

- Continuous generation of an essentially constant quantity of oxygen per time unit using an oxygen generation device;
- Transportation of the generated oxygen to a gas storage unit (19, 219, 319), which has at least one chamber (228, 328, 351, 352) with a variable volume that serves to hold a quantity of oxygen to be stored, wherein the chamber (228, 328, 351, 352) is connected to a gas outlet of the oxygen generation device via a gas supply pipe (226) that enters into the chamber (228, 328, 351, 352), and wherein the chamber (228, 328, 351, 352) has a gas outlet which is connected to a gas inlet of a digestion reactor (4, 204) particularly by means of a control unit (20, 220, 320) for the purpose of the flow regulation of at least an oxygen current flowing from the gas storage unit (19, 219, 319) to the digestion reactor (4, 204);
- Taking of a quantity of oxygen currently needed from the gas storage unit (19, 219, 319) and transportation of the quantity of oxygen taken to the digestion reactor (4, 204) and/or to one or more other consumers;
- Digestion of the sample in the digestion reactor (4, 204) and introduction of the oxygen taken from the gas storage unit into the digestion reactor (4, 204); and
- Measurement of a measured value that depends on the measured variable in a gas current directed out of the digestion reactor (4, 204).

13. Procedure as claimed in Claim 12, further comprising :

- Transportation of the generated oxygen to the chamber (228) by means of a pump (212), wherein a wall section of the chamber (228) is deflected against a reset force when a quantity of oxygen contained in the chamber (228) increases while the volume of the chamber (228) also increases,

such that a volume of the generated oxygen is stored in the chamber (228) under a pressure determined by the reset force, said volume being dependent on a delivery rate at which the generated oxygen is transported.

14. Procedure as claimed in Claim 12,
wherein the gas storage unit (319) comprises at least two chambers (328, 351, 352), whose volume can be modified by means of a controller, wherein said procedure further comprises the following steps:

- Transportation of the generated oxygen to a first of the two chambers (328, 351, 352) while increasing the volume of the chamber (328, 351, 352) until a predefined volume and a predefined regulation pressure are reached in the chamber (328, 351, 352); and
- Once the predefined volume and the predefined regulation pressure have been reached in the first chamber, the quantity of oxygen currently required is taken from the first chamber.

15. Procedure as claimed in Claim 14,
wherein, while the quantity of oxygen currently required is taken from the first chamber, the oxygen generated by the oxygen generation device is transported to the other, second chamber of the least two chambers (328, 351, 352).

## Revendications

1. Analyseur (100) destiné à la détermination d'une grandeur mesurée dépendant de la concentration d'un ou de plusieurs constituants d'un échantillon, lequel appareil comprend :

- un réacteur de digestion (4, 204) avec une entrée de gaz (13) et une sortie de gaz (14) ;
- un dispositif de mesure, qui est relié à la sortie de gaz (14) du réacteur de digestion (4) ;
- un dispositif de contrôle et d'évaluation (17), qui est conçu pour recevoir et traiter les valeurs mesurées enregistrées par le dispositif de mesure et dépendant de la grandeur mesurée ;
- un dispositif de génération d'oxygène ; et
- un système de conduite de gaz, qui relie le dispositif de génération d'oxygène à l'entrée de gaz (13) du réacteur de digestion (4, 204), afin d'alimenter le réacteur de digestion (4, 204) en oxygène généré par le dispositif de génération d'oxygène,

**caractérisé en ce que** le système de conduite de gaz comprend un dispositif de stockage de gaz (19, 219, 319), lequel dispositif comprend au moins une chambre (228, 328, 351, 352) à volume variable servant à recevoir une quantité d'oxygène à stocker.

2. Analyseur (100) selon la revendication 1,
pour lequel le système de conduite de gaz comprend un dispositif de commande (20, 220, 320) en vue de la régulation du flux d'au moins un courant d'oxygène s'écoulant à travers le système de conduite de gaz depuis le dispositif de stockage de gaz (19, 219, 319) vers le réacteur de digestion (4).

3. Analyseur (100) selon la revendication 1 ou 2,
pour lequel une conduite d'alimentation en gaz (226) débouche dans la chambre (228, 328, 351, 352) du dispositif de stockage de gaz, laquelle conduite d'alimentation en gaz est reliée, notamment via une pompe (212, 312), à une sortie de gaz du dispositif de génération d'oxygène, et
pour lequel la chambre (228, 328, 351, 352) présente une sortie de gaz qui est reliée, notamment via le dispositif de commande (20, 220, 320), à l'entrée de gaz du réacteur de digestion (4, 204).

4. Analyseur (100) selon la revendication 2 ou 3,
pour lequel le système de conduite de gaz relie en outre le dispositif de génération d'oxygène à un ou plusieurs autres consommateurs via le dispositif de stockage de gaz (219, 319) pour fournir auxdits consommateurs l'oxygène généré par le dispositif de génération d'oxygène, et
pour lequel le dispositif de commande (220, 320) est en outre conçu pour commander un débit d'oxygène à travers le système de conduite de gaz depuis le dispositif de stockage de gaz (219, 319) vers un ou plusieurs autres consommateurs.

5. Analyseur (100) selon la revendication 4,
pour lequel la sortie de gaz d'au moins une chambre (228, 328, 351, 352) du dispositif de stockage de gaz (219, 319) est reliée via le dispositif de commande (220, 320) à l'autre consommateur ou à l'un ou à plusieurs, notamment à chacun, des autres consommateurs.

6. Analyseur (100) selon l'une des revendications 1 à 5,
pour lequel une conduite d'alimentation en gaz (226) débouche dans la chambre (228) et est reliée à une sortie de gaz du dispositif de génération d'oxygène, et pour lequel la chambre (228) comprend une partie de paroi configurée, laquelle est conçue pour être déviée contre une force de rappel lors d'une augmentation de la quantité d'oxygène contenue dans la chambre, augmentant ainsi le volume de la chambre (228).

7. Analyseur (100) selon l'une des revendications 1 à 6,
pour lequel la chambre (228) du dispositif de stockage de gaz (219) est formée par un cylindre (223) fermé sur un côté par un fond (224) et un piston (225) guidé dans le cylindre (223) de manière à être mobile axialement par rapport à l'axe du cylindre et fermant le cylindre (223) de manière étanche aux gaz sur son côté opposé au fond (224), et pour lequel la position axiale du piston (225) dépend de la quantité d'oxygène contenue dans la chambre (228).

8. Analyseur (100) selon la revendication 7, pour lequel le système de conduite de gaz comprend des moyens pour surveiller la position axiale du piston (225).

9. Analyseur (100) selon l'une des revendications 1 à 5, pour lequel le dispositif de stockage de gaz (319) comporte au moins deux chambres (328, 351, 352) dont le volume peut être modifié au moyen d'une commande, une conduite d'alimentation en gaz débouchant dans chacune des chambres (328, 351, 352), laquelle conduite peut être reliée à la sortie de gaz du dispositif de génération d'oxygène, et chacune des chambres (328, 351, 352) présentant une sortie de gaz, laquelle sortie est reliée, notamment par l'intermédiaire du dispositif de commande (320), en vue de la régulation du débit, à l'entrée de gaz du réacteur de digestion.

10. Analyseur (100) selon la revendication 9, pour lequel chacune des conduites d'alimentation en gaz est reliée à la sortie de gaz du dispositif de génération d'oxygène via un ensemble de vannes (350), et pour lequel l'ensemble de vannes (350) est conçu pour bloquer ou libérer sélectivement les conduites de gaz menant de la sortie de gaz du dispositif de génération d'oxygène aux chambres (328, 351, 352), notamment alternativement ou selon une séquence prédéfinie par le système de commande.

11. Analyseur (100) selon la revendication 9 ou 10, pour lequel les au moins deux chambres (328, 351, 352) sont formées chacune par un cylindre (323, 331, 336) fermé sur un côté par un fond (324, 334, 337) et par un piston (325, 333, 340) qui est mobile axialement dans le cylindre (323, 331, 336) par rapport à l'axe du cylindre et qui ferme le cylindre de manière étanche aux gaz, et pour lequel chaque piston (325, 333, 340) est relié à un entraînement (341, 342, 343) qui effectue un mouvement axial du piston (325, 333, 340) et peut notamment être actionné par le système de commande.

12. Procédé destiné à la détermination d'une grandeur mesurée dépendant de la concentration d'un ou de plusieurs constituants d'un échantillon, notamment avec un analyseur (100) selon l'une des revendications 1 à 11, lequel procédé comprend les étapes suivantes :

- Génération en continu d'une quantité pour l'essentiel constante d'oxygène par unité de temps au moyen d'un dispositif de génération d'oxygène ;
- Transport de l'oxygène généré dans un dispositif de stockage de gaz (19, 219, 319), lequel dispositif comporte au moins une chambre (228, 328, 351, 352) à volume variable servant à recevoir une quantité d'oxygène à stocker, la chambre (228, 328, 351, 352) étant reliée à une sortie de gaz du dispositif de génération d'oxygène via une conduite d'alimentation en gaz (226) débouchant dans la chambre (228, 328, 351, 352), et la chambre (228, 328, 351, 352) comportant une sortie de gaz qui, notamment par l'intermédiaire d'un dispositif de commande (20, 220, 320), est reliée à une entrée de gaz d'un réacteur de digestion (4, 204) en vue de la régulation du flux d'au moins un courant d'oxygène s'écoulant du dispositif de stockage de gaz (19, 219, 319) vers le réacteur de digestion (4, 204) ;
- Prélèvement d'une quantité d'oxygène actuellement nécessaire dans le dispositif de stockage de gaz (19, 219, 319) et transport de la quantité d'oxygène prélevée dans le réacteur de digestion (4, 204) et/ou vers un ou plusieurs autres consommateurs ;
- Réalisation d'une digestion de l'échantillon dans le réacteur de digestion (4, 204) et introduction dans le réacteur de digestion (4, 204) de l'oxygène prélevé à partir du dispositif de stockage de gaz ; et
- Mesure d'une valeur dépendant de la grandeur mesurée dans un courant de gaz évacué du réacteur de digestion (4, 204).

13. Procédé selon la revendication 12, comprenant en outre :

- Transport de l'oxygène généré dans la chambre (228) au moyen d'une pompe (212), une partie de la paroi de la chambre (228) étant déviée contre une force de rappel lors d'une augmentation d'une quantité d'oxygène contenue dans la chambre (228) tout en augmentant un volume de la chambre (228),

de telle sorte qu'un volume de l'oxygène généré est stocké dans la chambre (228) sous une pression déterminée par la force de rappel en fonction d'un débit auquel l'oxygène généré est transporté.

14. Procédé selon la revendication 12, pour lequel le dispositif de stockage de gaz (319) comprend au moins deux chambres (328, 351, 352), dont le volume est variable au moyen d'une commande, lequel procédé comprend en outre les étapes suivantes :

- Transport de l'oxygène généré dans une première des deux chambres (328, 351, 352) tout en augmentant le volume de la chambre (328, 351, 352) jusqu'à ce qu'un volume prédéfini et une pression de régulation prédéfinie soient atteints dans la chambre (328, 351, 352) ; et
- Après atteinte du volume prédéfini et de la pression de régulation prédéfinie dans la première chambre, prélèvement de la quantité d'oxygène actuellement nécessaire à partir de la première chambre.

15. Procédé selon la revendication 14, pour lequel, pendant le prélèvement de la quantité d'oxygène actuellement requise de la première chambre, l'oxygène généré par le dispositif de génération d'oxygène est transporté vers l'autre, deuxième chambre, parmi les au moins deux chambres (328, 351, 352).

Fig. 1

Fig. 2

Ø Zyl.

230

201

213

212

$F_{G,Kolben}$

225

223

kontinuierlicher
$O_2$-Fluss

221

208

$p_{Zyl.}$

227

diskontinuierlicher
Verbraucher

209

226

228

224

220

204

229

219

214

Fig. 3

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 989401 A2
- DE 69228519 T2
- DE 102008013754 A1
- DE 202013105594 U1
- DE 202012102724 U1